# EUROPEAN PATENT APPLICATION

(11) **EP 4 631 492 A1**
(43) Date of publication of application: **15.10.2025**
(21) Application number: 23900749.5
(22) Date of filing: 08.12.2023
(51) Int. Cl.: A61K 8/891, A61K 8/19, A61K 8/27, A61K 8/28, A61K 8/29, A61K 8/894, A61Q 1/02, A61Q 1/04, A61Q 1/10, A61Q 3/02, A61Q 17/04

(54) **COSMETIC**

(30) Priority: 08.12.2022 JP 2022196466
(71) Applicant: Momentive Performance Materials Japan LLC, Tokyo 107-6119 (JP)
(72) Inventor: HORIE Yutaka, Tokyo 107-6119 (JP); LI Qinghua, Tokyo 107-6119 (JP)
(74) Representative: Gille Hrabal Partnerschaftsgesellschaft mbB Patentanwälte
(86) International application number: PCT/JP2023/044036
(87) International publication number: WO 2024/122637

(57) **Abstract**

[Problem to be solved] To provide a cosmetic preparation with good dispersibility of fine metal oxide particles.

[Solution] A cosmetic preparation containing the following components (A), (B), and (C): (A) one or two or more selected from 1,1,1,3,5,5,5-heptamethyl-3-alkyl (C10-14) trisiloxanes; (B) fine metal oxide particles; and (C) a silicone oil with a number average molecular weight of 400 or more and less than 30,000 (excluding component (A)).

## Description

### Field of the Invention

The present invention relates to a cosmetic preparation that can be used as foundations, cosmetic primers, sunscreens, lipsticks, nail polishes, face powders, and others.

### Background of the Invention

In recent years, the demands on cosmetic preparations have continued to diversify, and in response thereto, their product forms have also become wide-ranging, including not only elements in terms of an item lineup such as foundations, sunscreens, lipsticks, nail polishes, face powders, and others, but also those in terms of forms such as liquid form, powder form, solid form, emulsion form, and others as subcategories of the above.

While pigments or metal oxide sunscreening agent powders are essential for many of these cosmetic preparations, the problem is how to improve the dispersibility of such powders in the various forms of cosmetic preparations. Thus, using surface-treated powders or using lipophilic surfactants has been known as a technique for improving the dispersibility of powders.

JP-A 2013-139400, JP-A 2004-182729, JP-A 2015-124220, and JP-A 2016-210744 discuss powders whose surface is treated with various types of surface treatment agents to improve the dispersibility of the powders or adjust their feels.

JP-A H3-264510, JP-A 2003-012466, JP-A 2003-146833, and JP-A 2003-226631 discuss improving the dispersibility of powders by using alkyl-modified trisiloxanes including 1,1,1,3,5,5,5-heptamethyl-3-dodecyltrisiloxane as lipophilic surfactants.

### Summary of the Invention

The present invention relates to a cosmetic preparation that attains excellent dispersibility of fine metal oxide particles, ease of formulation, and excellent temporal stability.

In the aforementioned conventional arts, using the surface-treated powders or using the lipophilic surfactants could not provide a solution to improvement in the dispersibility of powders as a component of cosmetic preparations.

The present inventors have found that the effect of improving the dispersibility of fine metal oxide particles can be increased by the combined use of a 1,1,1,3,5,5,5-heptamethyl-3-alkyl (C10-14) trisiloxane and a silicone oil having a number average molecular weight of 400 or more and less than 30,000 excluding the 1,1,1,3,5,5,5-heptamethyl-3-alkyl (C10-14) trisiloxane to reach the completion of the present invention.

In other words, the present invention is a cosmetic preparation containing the following components (A), (B), and (C):
(A) one or two or more selected from 1,1,1,3,5,5,5-heptamethyl-3-alkyl (C10-14) trisiloxanes;
(B) fine metal oxide particles; and
(C) a silicone oil with a number average molecular weight of 400 or more and less than 30,000 (excluding component (A)).

The cosmetic preparation of the present invention attains excellent temporal stability of the cosmetic preparation and suppresses the separation of contained components as the dispersibility of the component (B) is improved by the combined use of the components (A) and (C). Further, improving the dispersibility of the component (B) is preferable as a UV protection effect, which is one of the effects of containing the component (B), can also be increased.

Further, the cosmetic preparation of the present invention can control the UV protection effect by adjusting the content of the component (B) as the dispersibility of the component (B) is improved.
Further, the cosmetic preparation of the present invention can attain good smoothness or good spreading during use and provide a fine appearance by adjusting the contents of the components (A) and (C) according to the content of the component (B).

### Embodiments of the Invention

The component (A) may be one or two or more selected from 1,1,1,3,5,5,5-heptamethyl-3-alkyl (C10-14) trisiloxanes. Further, the component (A) may be in oil form at ordinary temperatures.

The component (A) is considered to be a component that acts to increase the dispersibility of the component (B) in the cosmetic preparation when used together with the component (C).

Non-limiting examples of the component (A) can include (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

The above components (A-1), (A-2), and (A-3) can be each used alone as the component (A). If the component (A) is used as a single component, the component (A-2) is preferably used.

A mixture of the components (A-1) and (A-2), a mixture of the components (A-1) and (A-3), a mixture of the components (A-2) and (A-3), or a mixture of the components (A-1), (A-2), and (A-3) can be used as the component (A).

If the component (A) is used as the mixture of the components (A-1) and (A-2) or the mixture of the components (A-2) and (A-3), a content of the component (A-2) contained in 100 mass% of the mixture is preferably 50 mass% or more, more preferably 60 mass% or more, and further preferably 70 mass% or more.

If the component (A) is used as the mixture of the components (A-1) and (A-3), an amount of the component (A-1) in a total amount of 100 mass% of the components (A-1) and (A-3) is preferably 20 to 80 mass%, more preferably 30 to 70 mass%, and further preferably 40 to 60 mass%. Further, in this case, an amount of the component (A-3) in a total amount of 100 mass% of the components (A-1) and (A-3) is preferably 80 to 20 mass%, more preferably 70 to 30 mass%, and further preferably 60 to 40 mass%.

If the component (A) is used as the mixture of the components (A-1), (A-2), and (A-3), an amount of the component (A-1) in a total of 100 mass% of the components (A-1), (A-2), and (A-3) is preferably 5 to 80 mass%, more preferably 10 to 70 mass%, and further preferably 20 to 60 mass%. Further, in this case, an amount of the component (A-2) in a total of 100 mass% of the components (A-1), (A-2), and (A-3) is preferably 10 to 70 mass%, more preferably 15 to 60 mass%, and further preferably 20 to 50 mass%. Further, in this case, an amount of the component (A-3) in a total of 100 mass% of the components (A-1), (A-2), and (A-3) is preferably 10 to 70 mass%, more preferably 15 to 60 mass%, and further preferably 20 to 50 mass%.

Non-limiting examples of the fine metal oxide particles of the component (B) can include fine iron oxide particles, fine titanium oxide particles, fine zinc oxide particles, fine zirconium oxide particles, fine cerium oxide particles, or combinations of these, and others.

The shape of the fine metal oxide particles of the component (B) is not particularly limited, and can be, for example, spherical or plate-like, or a regular shape or an irregular shape. Further, the fine metal oxide particles of the component (B) may be hydrophilically or hydrophobically treated.

As the fine metal oxide particles of the component (B), those used in common cosmetic preparations containing fine metal oxide particles can be used without any particular limitation. Further, the fine metal oxide particles of the component (B) may have an average particle size of 10 µm or less, as measured, for example, by a laser diffraction scattering method. A lower limit of the average particle size of the fine metal oxide particles of the component (B) is not particularly limited, but may be about 0.1 µm in usual.

The fine metal oxide particles of the component (B) may be a component having the function of producing a UV protection effect. If the component (B) is a component having the function of producing a UV protection effect, the level of the UV protection effect (for example, UV protection level evaluated by SPF rating) can be controlled by adjusting (increasing or reducing) the content of the component (B) in the cosmetic preparation.

As the fine iron oxide particles, pigments such as iron oxide red, ocher, magnetite, or combinations of these, and others may be used without limitation. Further, they can be used after hydrophobic surface treatments.

The fine titanium oxide particles can be produced by a method such as the sulfuric acid method, the chlorine method, or the like, or commercially available products can also be used. Non-limiting examples of the commercially available fine titanium oxide particles can include the SIV series, TTO-55 series, and TTO-S series (which are manufactured by ISHIHARA SANGYO KAISHA, LTD.), MT-100TV, MT-500V, and MT-01 (which are manufactured by TAYCA Co., Ltd.), and others.

The fine titanium oxide particles can be used after hydrophilic or hydrophobic surface treatments as necessary. Examples of the hydrophilic treatment techniques can include surface treatments such as treatments with polyhydric alcohols such as glycerin or the like, treatments with hyaluronic acid, and others. Examples of the hydrophobic treatment techniques can include surface treatments such as fluorine compound treatments, silicone treatments, alkyl treatments, alkylsilane treatments, metallic soap treatments, water-soluble polymer treatments, amino acid treatments, N-acyl amino acid treatments, lecithin treatments, organic titanate treatments, polyol treatments, acrylic resin treatments, methacrylic resin treatments, urethane resin treatments, and others.

The fine titanium oxide particles or surface-treated fine titanium oxide particles can be combined into the powder-containing cosmetic preparation as they are. As necessary, they can be used by applying as a coating to the surface of another powder such as a plate-like powder such as talc, mica, sericite, or the like, silica, a spherical powder, or the like.

Applying the fine titanium oxide particles as a coating to a plate-like powder or a spherical powder is preferable as the fine titanium oxide particles are less likely to aggregate so that a high UV protection effect can be attained. Further, applying the fine titanium oxide particles as a coating to a plate-like powder or a spherical powder is preferable as squeakiness specific to the fine titanium oxide particles is less likely to be felt during use so that the cosmetic preparation can attain a good feel during use.

The shape of the fine zinc oxide particles is not particularly limited. Commercially available products can be used as the fine zinc oxide particles. Non-limiting examples of the commercially available products can include FINEX-25, FINEX-30, and FINEX-50 (which are manufactured by SAKAI CHEMICAL INDUSTRY CO., LTD.), MZ-300 and MZ-500 (which are manufactured by TAYCA Co., Ltd.), and others.

The fine zinc oxide particles can be used after hydrophilic or hydrophobic surface treatments as necessary. Examples of the hydrophilic treatment techniques can include surface treatments such as treatments with polyhydric alcohols such as glycerin or the like, treatments with hyaluronic acid, and others. Examples of the hydrophobic treatment techniques can include surface treatments such as fluorine compound treatments, silicone treatments, alkyl treatments, alkylsilane treatments, metallic soap treatments, water-soluble polymer treatments, amino acid treatments, N-acyl amino acid treatments, lecithin treatments, organic titanate treatments, polyol treatments, acrylic resin treatments, methacrylic resin treatments, urethane resin treatments, and others.

The fine zinc oxide particles or surface-treated fine zinc oxide particles can be combined into the powder-containing cosmetic preparation as they are. Further, as necessary, they can be used by applying as a coating to the surface of another powder such as a plate-like powder such as talc, mica, sericite, or the like, silica, a spherical powder, or the like.

Applying the fine zinc oxide particles as a coating to a plate-like powder or a spherical powder is preferable as the fine zinc oxide particles are less likely to aggregate so that a high UV protection effect can be attained. Further, applying the fine zinc oxide particles as a coating to a plate-like powder or a spherical powder is preferable as squeakiness specific to the fine zinc oxide particles is less likely to be felt during use so that the cosmetic preparation can attain a good feel during use.

The component (C) is a silicone oil with a number average molecular weight of 400 or more and less than 30,000, and excludes components qualifying as the component (A).

The number average molecular weight of the component (C) may be measured by a publicly-known measurement method such as a gel permeation chromatography method, a liquid chromatography method, a gas chromatography method, a static light scattering method, or the like. In general, it may be measured by a gel permeation chromatography method, which may use toluene as the solvent and polystyrene as the standard. If the molecular weight is below 1000 or the like, a gas chromatography method may be used.

Many of silicone oils with a number average molecular weight of less than 400 are relatively highly volatile and may volatilize during use to reduce the dispersibility of powders. Further, those with a number average molecular weight of 30,000 or more are highly viscous and may reduce the dispersibility of each powder. Accordingly, a silicone oil with a number average molecular weight of 400 or more and less than 30,000 is required as the component (C). In view of good dispersibility of powders, the component (C) is preferably a silicone oil with a number average molecular weight of 400 or more and less than 25,000, and further preferably a silicone oil with a number average molecular weight of 400 or more and less than 20,000.

The component (C) is a component that acts to increase the dispersibility of the component (B) in the cosmetic preparation when used together with the component (A). Improving the dispersibility of the component (B) in the cosmetic preparation is preferable as a UV protection effect, which is one of the effects of containing the component (B), can also be increased.

In one non-limiting embodiment, the silicone oil of the component (C) may have a boiling point or a thermal decomposition temperature of 220°C or more. The boiling point or thermal decomposition temperature of the component (C) may be measured at atmospheric pressure.

The silicone oil of the component (C) is preferably represented by the following average composition formula:

RₐSiO_{(4-a)/2}

wherein R is a substituted or unsubstituted monovalent hydrocarbon group, the monovalent hydrocarbon groups may be the same or different, and a represents a positive number less than 4.

Examples of the substituted or unsubstituted monovalent hydrocarbon group of R can include, for example, a group having 1 to 12, particularly 1 to 8 carbons in the portion excluding a substituent. Non-limiting examples of the substituted or unsubstituted monovalent hydrocarbon group of R can include an alkyl group such as a methyl group, an ethyl group, a hexyl group, a decyl group, or the like, an aryl group such as a phenyl group, or the like, an aralkyl group such as 2-methylphenyl ethyl group, or the like, an amino-containing group such as a 3-aminopropyl group, a 2-aminoethyl-3-aminopropyl group, or the like, a polyoxyalkylene group such as a polyoxyethylene group, a polyoxypropylene group, a polyoxyethylene-polyoxypropylene group, or the like, and others.

The component (C) preferably has a surface tension of 30 mN/m or less from the viewpoint of improving the dispersibility of the fine metal oxide particles of the component (B) together with the component (A). The component (C) has a surface tension of preferably 30 mN/m or less and particularly preferably 25 mN/m or less, as measured, for example, by the plate method (Wilhelmy method) or the ring method (du Noüy method).

The silicone oil of the component (C) is more preferably a polydimethylsiloxane, a polydimethylsiloxane-polydiphenylsiloxane copolymer, a polymethyltrimethylsilylsiloxane-polydiphenylsiloxane copolymer, a trimethylsilyl-terminated mono or polytrimethylsilylphenylsiloxane, a polyether-modified oil, or a combination of these, or the like.

Non-limiting examples of the polyether-modified oil can include a polyoxyethylene-modified silicone oil, a polyoxyethylene-polyoxypropylene-modified silicone oil, a polyoxypropylene-modified silicone oil, or a combination of these.

A mass ratio of a content of the component (A) to a content of the component (C) ((A)/(C)) is preferably in a range of 0.1 to 5, 0.15 to 3, and further, more preferably in a range of 0.15 to 2 from the viewpoint of increasing the dispersibility of the component (B) in the cosmetic preparation.

The cosmetic preparation of the present invention contains the components (A), (B), and (C) in an amount of preferably 3 to 85 mass%, more preferably 5 to 80 mass%, and further preferably 20 to 70 mass% in total.

In the cosmetic preparation of the present invention, the contents of the components (A) and (C) can be adjusted with the content of the component (B) as a reference value as the dispersibility of the component (B) is improved by the combined use of the components (A) and (C).

The contents of the components (A) and (C) with respect to the component (B) can vary depending on multiple factors such as the physical properties and chemical properties required of the cosmetic preparation, the type of the cosmetic preparation (for example, a skin cosmetic preparation), and others. For example, when the content of the component (B) is 100 parts by mass, the content of the component (A) is preferably 20 to 800 parts by mass, more preferably 20 to 700 parts by mass, and further preferably 20 to 600 parts by mass. When the content of the component (B) is 100 parts by mass, the content of the component (C) is preferably 20 to 1500 parts by mass, more preferably 20 to 1300 parts by mass, and further preferably 20 to 1200 parts by mass.

In addition to the components (A), (B), and (C), various types of raw materials which are commonly used in cosmetic preparations may be added to the cosmetic preparation of the present invention depending on the type or the formulation of the cosmetic preparation. For example, each of the following components can be appropriately selected and contained in the cosmetic preparation of the present invention.

The cosmetic preparation of the present invention can contain an oily component, and examples include, but are not limited to, solid paraffin, ceresin, beeswax, carnauba wax, wood wax, candelilla wax, stearic acid, cetanol, cholesterol, microcrystalline wax, petroleum jelly, lanolin, partially hydrogenated oils, squalene, liquid paraffin, castor oils, diglycerides, triglycerides, higher fatty acids, higher alcohols, perfluoropolyethers, perfluorodecalin, perfluorooctane, ester oils, octyldodecyl myristate, octyldodecyl oleate, neopentyl glycol dioctanoate, or combinations of these, and others.

The cosmetic preparation of the present invention can contain an aqueous component, and examples include, but are not limited to, glycerin, sorbitol, propylene glycol, 1,3-butylene glycol, polyethylene glycol, carboxyvinyl polymer, cellulose derivatives, sodium alginate, ethanol, or combinations of these, and others.

The cosmetic preparation of the present invention can contain a surfactant, and examples include, but are not limited to, one or a combination of two or more selected from the following groups,
the following anionic emulsifiers: sodium alkyl benzene sulfonates such as sodium hexyl benzene sulfonate, sodium octyl benzene sulfonate, sodium decyl benzene sulfonate, sodium dodecyl benzene sulfonate, sodium cetyl benzene sulfonate, and sodium myristyl benzene sulfonate; sodium alkyl naphthyl sulfonates such as sodium butyl naphthyl sulfonate; polyoxyethylene monoalkyl ether sulfate sodium salts such as sodium polyoxyethylene octyl ether sulfate, sodium polyoxyethylene decyl ether sulfate, and sodium polyoxyethylene icosyl ether sulfate; polyoxyethylene mono(alkylphenyl)ether sulfate sodium salts such as sodium polyoxyethylene decylphenyl ether sulfate; or combinations of these, and others,
the following cationic emulsifiers: quaternary ammonium salts such as octyl trimethyl ammonium chloride, dodecyl trimethyl ammonium chloride, hexadecyl trimethyl ammonium chloride, beef tallow trimethyl ammonium chloride, coconut oil trimethyl ammonium chloride, octyl dimethyl benzyl ammonium chloride, decyl dimethyl benzyl ammonium chloride, and dioctadecyl dimethyl ammonium chloride; or combinations of these, and others, and
the following nonionic emulsifiers: glycerin fatty acid esters such as glyceryl monolaurate, glyceryl monomyristate, glyceryl monostearate, and glyceryl monooleate; polyglycerin fatty acid esters, sorbitan fatty acid esters, polyoxyethylene glycerin fatty acid esters, polyoxyethylene sorbitan fatty acid esters, and polyoxyethylene fatty acid esters having a lauric acid residue, a myristic acid residue, a stearic acid residue, or an oleic acid residue; polyoxyethylene alkyl ethers such as polyoxyethylene lauryl ether, polyoxyethylene cetyl ether, polyoxyethylene stearyl ether, and polyoxyethylene oleyl ether; or combinations of these, and others.

The cosmetic preparation of the present invention can contain other components in the range that the problem or the effects of the present invention are not impaired, and examples include, but are not limited to, coloring agents such as organic pigments, organic dyes, and others, preservatives, antioxidants, colorants, thickeners, pH adjusters, fragrances, UV absorbers, moisturizers, blood circulation promoters, cooling agents, antiperspirants, bactericides, skin activators, and others.

Further, the cosmetic preparation of the present invention can also contain a desired amount of water depending on the type or the formulation of the cosmetic preparation.

The cosmetic preparation of the present invention is preferably a skin cosmetic preparation selected from foundations, cosmetic primers, sunscreens, lipsticks, nail polishes, eye shadows, and face powders.

The cosmetic preparation of the present invention encompasses each of the following inventions.
(1) A cosmetic preparation containing the following components (A), (B), and (C):
   (A) one or two or more selected from 1,1,1,3,5,5,5-heptamethyl-3-alkyl (C10-14) trisiloxanes;
   (B) fine metal oxide particles; and
   (C) a silicone oil with a number average molecular weight of 400 or more and less than 30,000 (excluding component (A)).
(2) The cosmetic preparation according to (1), wherein the component (A) is one or two or more selected from the group composed of (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(3) The cosmetic preparation according to (1) or (2), wherein the component (A) is one component selected from the group composed of (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(4) The cosmetic preparation according to (1) or (2), wherein the component (A) is two components selected from the group composed of (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(5) The cosmetic preparation according to (1) or (2), wherein the component (A) includes three components (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(6) The cosmetic preparation according to (1) or (2), wherein the component (A) is two components selected from the group composed of (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, the two components are the components (A-1) and (A-2) or the components (A-2) and (A-3), and a content of the component (A-2) is 50 mass% or more of the component (A).
(7) The cosmetic preparation according to (1) or (2), wherein the component (A) is two components selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, the two components are the components (A-1) and (A-2) or the components (A-2) and (A-3), and a content of the component (A-2) is 60 mass% or more of the component (A).
(8) The cosmetic preparation according to (1) or (2), wherein the component (A) is two components selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, the two components are the components (A-1) and (A-2) or the components (A-2) and (A-3), and a content of the component (A-2) is 70 mass% or more of the component (A).
(9) The cosmetic preparation according to (2), wherein the component (A) is used as a mixture of the components (A-1) and (A-3), and an amount of the component (A-1) is 20 to 80 mass% and an amount of the component (A-3) is 80 to 20 mass% in a total amount of 100 mass% of the components (A-1) and (A-3).
(10) The cosmetic preparation according to (2), wherein the component (A) is used as a mixture of the components (A-1) and (A-3), and an amount of the component (A-1) is 30 to 70 mass% and an amount of the component (A-3) is 70 to 30 mass% in a total amount of 100 mass% of the components (A-1) and (A-3).
(11) The cosmetic preparation according to (2), wherein the component (A) is used as a mixture of the components (A-1) and (A-3), and an amount of the component (A-1) is 40 to 60 mass% and an amount of the component (A-3) is 60 to 40 mass% in a total amount of 100 mass% of the components (A-1) and (A-3).
(12) The cosmetic preparation according to (2), wherein the component (A) is used as a mixture of the components (A-1), (A-2), and (A-3), and an amount of the component (A-1) is 5 to 80 mass%, an amount of the component (A-2) is 10 to 70 mass%, and an amount of the component (A-3) is 10 to 70 mass% in a total of 100 mass% of the components (A-1), (A-2), and (A-3).
(13) The cosmetic preparation according to (2), wherein the component (A) is used as a mixture of the components (A-1), (A-2), and (A-3), and an amount of the component (A-1) is 10 to 70 mass%, an amount of the component (A-2) is 15 to 60 mass%, and an amount of the component (A-3) is 15 to 60 mass% in a total of 100 mass% of the components (A-1), (A-2), and (A-3).
(14) The cosmetic preparation according to (2), wherein the component (A) is used as a mixture of the components (A-1), (A-2), and (A-3), and an amount of the component (A-1) is 20 to 60 mass%, an amount of the component (A-2) is 20 to 50 mass%, and an amount of the component (A-3) is 20 to 50 mass% in a total of 100 mass% of the components (A-1), (A-2), and (A-3).
(15) The cosmetic preparation according to any one of (1) to (14), wherein the fine metal oxide particles of the component (B) are selected from fine iron oxide particles, fine titanium oxide particles, fine zinc oxide particles, fine zirconium oxide particles, fine cerium oxide particles, or combinations of these.
(16) The cosmetic preparation according to any one of (1) to (14), wherein the fine metal oxide particles of the component (B) are selected from fine iron oxide particles, fine titanium oxide particles, fine zinc oxide particles, fine zirconium oxide particles, fine cerium oxide particles, or combinations of these, and are hydrophilically or hydrophobically treated.
(17) The cosmetic preparation according to any one of (1) to (16), wherein the fine metal oxide particles of the component (B) have an average particle size of primary particles of 100 nm or less and further 80 nm or less, and a lower limit of the average particle size is 30 nm.
(18) The cosmetic preparation according to any one of (1) to (17), wherein the fine metal oxide particles of the component (B) are fine iron oxide particles.
(19) The cosmetic preparation according to any one of (1) to (17), wherein the fine metal oxide particles of the component (B) are fine iron oxide particles, and the fine iron oxide particles are a pigment selected from iron oxide red, ocher, magnetite, or combinations of these.
(20) The cosmetic preparation according to any one of (1) to (17), wherein the fine metal oxide particles of the component (B) are fine iron oxide particles, and the fine iron oxide particles are hydrophobically treated.
(21) The cosmetic preparation according to any one of (1) to (17), wherein the fine metal oxide particles of the component (B) are fine titanium oxide particles.
(22) The cosmetic preparation according to any one of (1) to (17), wherein the fine metal oxide particles of the component (B) are fine titanium oxide particles, and the fine titanium oxide particles are applied as a coating to the surface of another powder selected from plate-like powders including talc, mica, and sericite, silica, and spherical powders.
(23) The cosmetic preparation according to any one of (1) to (17), wherein the fine metal oxide particles of the component (B) are fine titanium oxide particles, and the fine titanium oxide particles are hydrophilically or hydrophobically surface-treated.
(24) The cosmetic preparation according to any one of (1) to (17), wherein the fine metal oxide particles of the component (B) are fine titanium oxide particles, and the fine titanium oxide particles are hydrophilically surface-treated by a treatment with polyhydric alcohols including glycerin or a treatment with hyaluronic acid, or hydrophobically surface-treated by a treatment selected from fluorine compound treatments, silicone treatments, alkyl treatments, alkylsilane treatments, metallic soap treatments, water-soluble polymer treatments, amino acid treatments, N-acyl amino acid treatments, lecithin treatments, organic titanate treatments, polyol treatments, acrylic resin treatments, methacrylic resin treatments, and urethane resin treatments.
(25) The cosmetic preparation according to any one of (1) to (17), wherein the fine metal oxide particles of the component (B) are fine zinc oxide particles.
(26) The cosmetic preparation according to any one of (1) to (17), wherein the fine metal oxide particles of the component (B) are fine zinc oxide particles, and the fine zinc oxide particles are applied as a coating to the surface of another powder selected from plate-like powders including talc, mica, and sericite, silica, and spherical powders.
(27) The cosmetic preparation according to any one of (1) to (17), wherein the fine metal oxide particles of the component (B) are fine zinc oxide particles, and the fine zinc oxide particles are hydrophilically or hydrophobically surface-treated.
(28) The cosmetic preparation according to any one of (1) to (17), wherein the fine metal oxide particles of the component (B) are fine zinc oxide particles, and the fine zinc oxide particles are hydrophilically surface-treated by a treatment with polyhydric alcohols including glycerin or a treatment with hyaluronic acid, or hydrophobically surface-treated by a treatment selected from fluorine compound treatments, silicone treatments, alkyl treatments, alkylsilane treatments, metallic soap treatments, water-soluble polymer treatments, amino acid treatments, N-acyl amino acid treatments, lecithin treatments, organic titanate treatments, polyol treatments, acrylic resin treatments, methacrylic resin treatments, and urethane resin treatments.
(29) The cosmetic preparation according to any one of (1) to (28), wherein the silicone oil of the component (C) is represented by the following composition formula:

   RₐSiO_{(4-a)/2}

   wherein R is a substituted or unsubstituted monovalent hydrocarbon group, the monovalent hydrocarbon groups may be the same or different, and a represents a positive number less than 4.
(30) The cosmetic preparation according to any one of (1) to (29), wherein the silicone oil of the component (C) has a surface tension of 30 mN/m or less.
(31) The cosmetic preparation according to any one of (1) to (29), wherein the silicone oil of the component (C) is selected from polydimethylsiloxanes, polydimethylsiloxane-polydiphenylsiloxane copolymers, polymethyltrimethylsilylsiloxane-polydiphenylsiloxane copolymers, trimethylsilyl-terminated mono or polytrimethylsilylphenylsiloxanes, polyether-modified oils selected from polyoxyethylene-modified silicone oils, polyoxyethylene-polyoxypropylene-modified silicone oils, and polyoxypropylene-modified silicone oils, or combinations of these.
(32) The cosmetic preparation according to any one of (1) to (31), wherein a mass ratio of a content of the component (A) to a content of the component (C) ((A)/(C)) is in a range of 0.1 to 5.
(33) The cosmetic preparation according to any one of (1) to (31), wherein a mass ratio of a content of the component (A) to a content of the component (C) ((A)/(C)) is in a range of 0.15 to 3.
(3432) The cosmetic preparation according to any one of (1) to (31), wherein the preparation contains the components (A), (B), and (C) in an amount of 3 to 85 mass% in total.
(35) The cosmetic preparation according to any one of (1) to (31), wherein a total of the components (A), (B), and (C) is 5 to 80 mass%.
(36) The cosmetic preparation according to any one of (1) to (31), wherein when a content of the component (B) is 100 parts by mass, a content of the component (A) is 20 to 800 parts by mass, and a content of the component (C) is 20 to 1500 parts by mass.
(37) The cosmetic preparation according to any one of (1) to (31), wherein when a content of the component (B) is 100 parts by mass, a content of the component (A) is 20 to 700 parts by mass, and a content of the component (C) is 20 to 1300 parts by mass.
(38) The cosmetic preparation according to any one of (1) to (31), wherein when a content of the component (B) is 100 parts by mass, a content of the component (A) is 20 to 600 parts by mass, and a content of the component (C) is 20 to 1200 parts by mass.
(39) The cosmetic preparation according to any one of (1) to (38), wherein in addition to the components (A), (B), and (C), the preparation contains an oily component selected from solid paraffin, ceresin, beeswax, carnauba wax, wood wax, candelilla wax, stearic acid, cetanol, cholesterol, microcrystalline wax, petroleum jelly, lanolin, partially hydrogenated oils, squalene, liquid paraffin, castor oils, diglycerides, triglycerides, higher fatty acids, higher alcohols, perfluoropolyethers, perfluorodecalin, perfluorooctane, ester oils, octyldodecyl myristate, octyldodecyl oleate, neopentyl glycol dioctanoate, or combinations of these.
(40) The cosmetic preparation according to any one of (1) to (38), wherein in addition to the components (A), (B), and (C), the preparation contains an aqueous component selected from glycerin, sorbitol, propylene glycol, 1,3-butylene glycol, polyethylene glycol, carboxyvinyl polymer, cellulose derivatives, sodium alginate, ethanol, or combinations of these.
(41) The cosmetic preparation according to any one of (1) to (40), wherein in addition to the components (A), (B), and (C), the preparation contains, as a surfactant, one or a combination of two or more selected from the following groups,
   anionic emulsifiers selected from the following: sodium alkyl benzene sulfonates such as sodium hexyl benzene sulfonate, sodium octyl benzene sulfonate, sodium decyl benzene sulfonate, sodium dodecyl benzene sulfonate, sodium cetyl benzene sulfonate, and sodium myristyl benzene sulfonate; sodium alkyl naphthyl sulfonates such as sodium butyl naphthyl sulfonate; polyoxyethylene monoalkyl ether sulfate sodium salts such as sodium polyoxyethylene octyl ether sulfate, sodium polyoxyethylene decyl ether sulfate, and sodium polyoxyethylene icosyl ether sulfate; polyoxyethylene mono(alkylphenyl)ether sulfate sodium salts such as sodium polyoxyethylene decylphenyl ether sulfate; or combinations of these,
   cationic emulsifiers selected from the following: quaternary ammonium salts such as octyl trimethyl ammonium chloride, dodecyl trimethyl ammonium chloride, hexadecyl trimethyl ammonium chloride, beef tallow trimethyl ammonium chloride, coconut oil trimethyl ammonium chloride, octyl dimethyl benzyl ammonium chloride, decyl dimethyl benzyl ammonium chloride, and dioctadecyl dimethyl ammonium chloride; or combinations of these, and
   nonionic emulsifiers selected from the following: glycerin fatty acid esters such as glyceryl monolaurate, glyceryl monomyristate, glyceryl monostearate, and glyceryl monooleate; polyglycerin fatty acid esters, sorbitan fatty acid esters, polyoxyethylene glycerin fatty acid esters, polyoxyethylene sorbitan fatty acid esters, and polyoxyethylene fatty acid esters having a lauric acid residue, a myristic acid residue, a stearic acid residue, or an oleic acid residue; polyoxyethylene alkyl ethers such as polyoxyethylene lauryl ether, polyoxyethylene cetyl ether, polyoxyethylene stearyl ether, and polyoxyethylene oleyl ether; or combinations of these.
(42) The cosmetic preparation according to any one of (1) to (41), wherein in addition to the components (A), (B), and (C), the preparation contains a component selected from coloring agents including organic pigments and organic dyes, preservatives, antioxidants, colorants, thickeners, pH adjusters, fragrances, UV absorbers, moisturizers, blood circulation promoters, cooling agents, antiperspirants, bactericides, skin activators, water, or combinations of these.
(43) The cosmetic preparation according to any one of (1) to (42), wherein the preparation is in any of liquid formulations, powder formulations, solid formulations, and emulsion formulations.
(44) The cosmetic preparation according to any one of (1) to (42), wherein the preparation is a skin cosmetic preparation selected from foundations, cosmetic primers, sunscreens, lipsticks, eye shadows, nail polishes, and face powders.

### Examples

Examples of the present invention are illustrated below, but the present invention is not limited by these examples.

The components used in the examples and comparative examples are as follows. In the following, "synthetic product" means that the product was synthesized by the present inventors.

### Component (A)

1,1,1,3,5,5,5-heptamethyl-3-dodecyltrisiloxane: synthetic product
1,1,1,3,5,5,5-heptamethyl-3-decyltrisiloxane: synthetic product
1,1,1,3,5,5,5-heptamethyl-3-tetradecyltrisiloxane: synthetic product

### Comparative component of component (A)

1,1,1,3,5,5,5-heptamethyl-3-octyltrisiloxane: Silsoft 034 manufactured by Momentive Performance Materials Inc.

### Component (B)

Hydrophobically surface-treated titanium oxide/aluminum hydroxide: SI01-2 TiO₂ CR-50 manufactured by DAITO KASEI KOGYO CO., LTD.

Hydrophobically surface-treated zinc oxide: SI01-4 ZnO-300 manufactured by DAITO KASEI KOGYO CO., LTD.

Hydrophobically surface-treated iron oxide (yellow): SI-2 YELLOW LL-100P manufactured by DAITO KASEI KOGYO CO., LTD.

Hydrophobically surface-treated iron oxide (red): SI-2 RED R-516P manufactured by DAITO KASEI KOGYO CO., LTD.

Hydrophobically surface-treated iron oxide (black): SI-2 BLACK BL-100P manufactured by DAITO KASEI KOGYO CO., LTD.

### Component (C)

Polydimethylsiloxane (viscosity 5 mm²/s, molecular weight 680 (a value calculated from the structure), surface tension 19.7 mN/m): Element14 PDMS 5-JC manufactured by Momentive Performance Materials Inc.

### (Other components)

Ethylhexyl methoxycinnamate: Uvinul MC 80 N manufactured by BASF SE
Trimethylsiloxysilicate: SR 1000 manufactured by Momentive Performance Materials Inc.
Cyclopentasiloxane, (dimethicone/vinyl dimethicone) crosspolymer: SFE 839 gel manufactured by Momentive Performance Materials Inc.
Sorbitan stearate: NIKKOL SS-10V manufactured by Nikko Chemicals Co., Ltd.
Sorbitan sesquiisostearate: COSMOL 182V manufactured by The Nisshin OilliO Group, Ltd.
Glycerin: reagent
Polysorbate 20: NONION LT-20 manufactured by NOF CORPORATION
Sodium chloride: reagent
Phenoxy ethanol: reagent

### Examples 1 to 5 and comparative examples 1 to 3

Phase I components, phase II components, and phase III components shown in Table 1 were each stirred until the components became uniform, and then stirred at 70°C for 30 minutes to prepare a mixture of each phase. Next, the mixture of the phase II was added to the mixture of the phase I and stirred until they became uniform, and subsequently the mixture of the phase III was added thereto and stirred until they became uniform. After that, the mixture was stirred with a homogenizer at 2,000 rpm for 5 minutes to obtain a cosmetic primer.

Each of the following evaluation tests was conducted. The results are shown in Table 1. The numerical values relating to the components in Table 1 are expressed as mass%.

### (Feeling on the skin during application)

The feeling when an appropriate amount (about 1 g) of each of the cosmetic preparations of the examples and comparative examples was placed on the hand, and then applied to the face and spread with fingers was evaluated. Ten panelists made evaluations out of 5 with comparative example 1 as 3 (5: superior, 4: slightly superior, 3: equal, 2: slightly inferior, 1: inferior), and the average score thereof was as shown in Table 1.

### (Appearance after application)

Ten panelists visually observed and evaluated the condition after testing the feeling on the skin during application out of 5 with comparative example 1 as 3 (5: superior, 4: slightly superior, 3: equal, 2: slightly inferior, 1: inferior), and the average score thereof was as shown in Table 1.

### (Appearance after storage at 50°C for one month)

The appearance of each of the cosmetic preparations of the examples and comparative examples after storage in a glass container at 50°C for one month after production was visually observed.

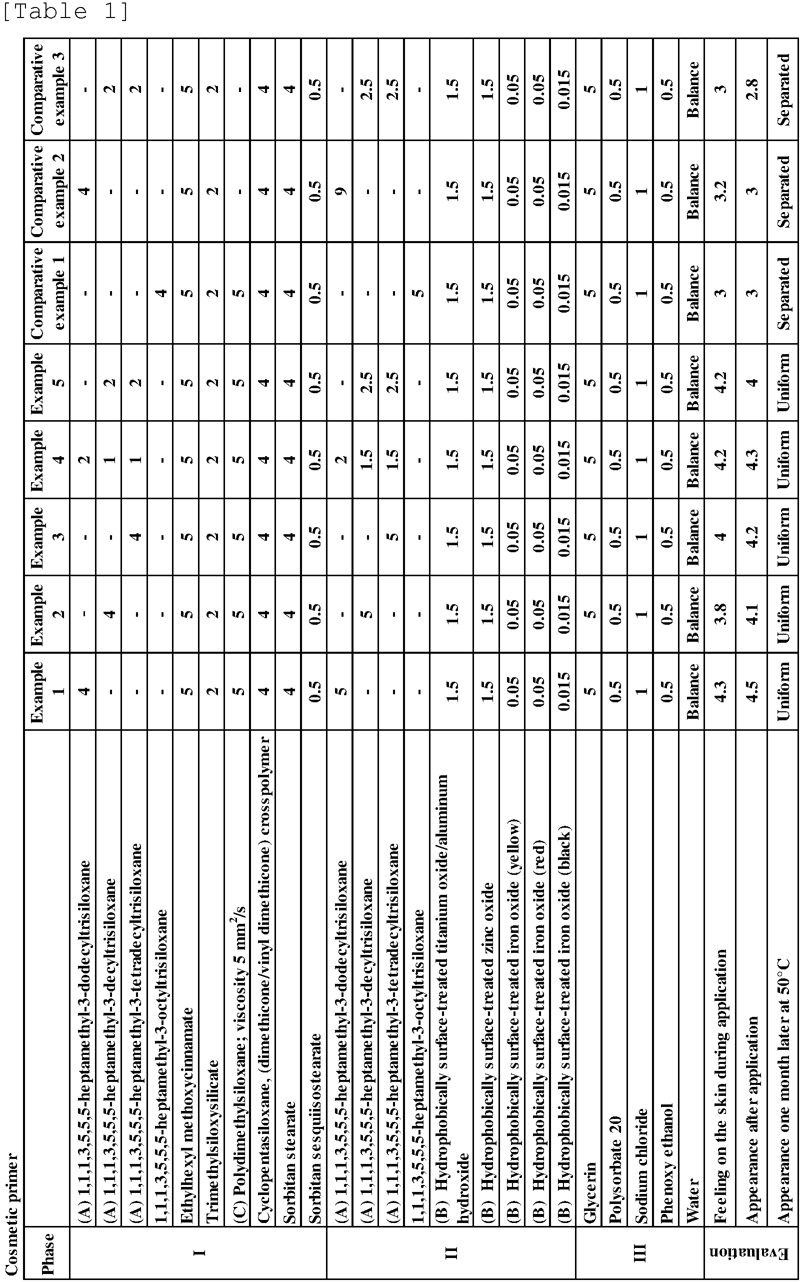

In the evaluation of the feeling on the skin during application and the appearance after application, the average evaluation scores of examples 1 to 5 were 3.8 or more, i.e., almost 4 or more, and those of comparative examples 1 to 3 were 3.2 or less, i.e., almost 3 or less. Further, the appearance one month later at 50°C in examples 1 to 5 was a non-separated, uniform appearance, but separation was observed in the appearance of comparative examples 1 to 3.

It could be confirmed from the comparison of examples 1 to 5 and comparative examples 1 to 3 that the dispersibility of the component (B) was improved by the combined use of the components (A) and (C).

### Example 6 (Liquid foundation)

| | | |
|---|---|---|
| **(1)** | **(A) 1,1,1,3,5,5,5-heptamethyl-3-dodecyltrisiloxane (synthetic product)** | **4.00** |
| **(2)** | **(C) SilShine 150* (surface tension 24.7 mN/m): diphenyl dimethicone molecular weight 700 (calculated value)** | **5.00** |
| **(3)** | **SilFonn Flexible Resin*: polymethylsilsesquioxane** | **1.00** |
| **(4)** | **SR 1000*: trimethylsiloxysilicate** | **1.00** |
| **(5)** | **Dodecamethylpentasiloxane (reagent)** | **2.00** |
| **(6)** | **(C) Velvesil DM LC gel*: dimethicone, cetearyl dimethicone crosspolymer** | **4.00** |
| **(7)** | **(C) HARMONIE Mesh emulsifier*: (caprylic/capric) triglyceride, PEG/PPG-20/15 dimethicone** | **5.00** |
| **(8)** | **(A) 1,1,1,3,5,5,5-heptamethyl-3-dodecyltrisiloxane (synthetic product)** | **7.00** |
| **(9)** | **HARMONIE Luxe 4 powder*: silica** | **5.00** |
| **(10)** | **(B) SI01-2CR-50 manufactured by DAITO KASEI KOGYO CO., LTD.: hydrophobically surface-treated titanium oxide** | **4.50** |
| **(11)** | **(B) SI-2 Yellow LL-100P manufactured by DAITO KASEI KOGYO CO., LTD.: hydrophobically surface-treated yellow pigment** | **0.28** |
| **(12)** | **(B) SI-2 Red R-516 manufactured by DAITO KASEI KOGYO CO., LTD.: hydrophobically surface-treated red pigment** | **0.18** |
| **(13)** | **(B) SI-2 Black BL-100P manufactured by DAITO KASEI KOGYO CO., LTD.: hydrophobically surface-treated black pigment** | **0.05** |
| **(14)** | **Water** | **54.99** |
| **(15)** | **Glycerin** | **5.00** |
| **(16)** | **Sodium chloride** | **1.00** |

| | | |
|---|---|---|
| **Those followed by an asterisk (*) are manufactured by Momentive Performance Materials Inc.** | | |

All the numerical values are expressed as mass%, and total up to 100 mass%. The indications (A), (B), and (C) mean that the components are those qualifying as the components (A), (B), and (C), respectively. For example, (7) contains a polyoxyethylene-polyoxypropylene-modified silicone oil.

### (Preparation method)

After the above components (1) to (7) and components (8) to (13) were each mixed until they became uniform, the uniform mixture of the components (1) to (7) and the uniform mixture of the components (8) to (13) were mixed at 70°C for 30 minutes to obtain a uniform mixture of the components (1) to (13). Subsequently, the components (14) to (16) uniformly dissolved at 70°C were added and mixed to the uniform mixture of the components (1) to (13), and then stirred with a homogenizer at 2,000 rpm for 5 minutes to obtain a liquid foundation.

### (Evaluation)

The obtained liquid foundation was a uniform dispersion without lumps or the like, was smooth and spread well when applied to the skin, and was felt soft and smooth after drying. Further, this liquid foundation was stable even one month later at 50°C.

### Example 7 (concealer)

| | | |
|---|---|---|
| **(1)** | **(A) 1,1,1,3,5,5,5-heptamethyl-3-dodecyltrisiloxane (synthetic product)** | **10.0** |
| **(2)** | **(C) Element14 PDMS 5-JC* (surface tension 19.7 mN/m, viscosity 5 cSt): polydimethylsiloxane molecular weight 680 (calculated value)** | **57.0** |
| **(3)** | **CCS102-JA Boron Nitride*: boron nitride** | **10.0** |
| **(4)** | **HARMONIE Luxe 4 powder*: silica** | **5.0** |
| **(5)** | **(B) SI01-2CR-50 manufactured by DAITO KASEI KOGYO CO., LTD.: hydrophobically surface-treated titanium oxide** | **4.0** |
| **(6)** | **(B) SI-2 Yellow LL-100P manufactured by DAITO KASEI KOGYO CO., LTD.: hydrophobically surface-treated yellow pigment** | **0.9** |
| **(7)** | **(B) SI-2 Red R-516 manufactured by DAITO KASEI KOGYO CO., LTD.: hydrophobically surface-treated red pigment** | **0.3** |
| **(8)** | **(B) SI-2 Black BL-100P manufactured by DAITO KASEI KOGYO CO., LTD.: hydrophobically surface-treated black pigment** | **0.1** |
| **(9)** | **PMWAX82 manufactured by NIKKO RICA CORPORATION: polyethylene, microcrystalline wax** | **7.0** |
| **(10)** | **UltimoPureBEESWAX manufactured by Croda International Plc: beeswax** | **5.0** |

| | | |
|---|---|---|
| **All the numerical values are expressed as mass%, and total up to 100 mass %.** **Those followed by an asterisk (*) are manufactured by Momentive Performance Materials Inc.** | | |

### (Preparation method)

After the above components (1) to (8) were mixed at 90 to 95°C until they became uniform, the components (9) and (10) were added and mixed until they became uniform, and after the mixture was poured into a container and cooled, a concealer was obtained.

### (Evaluation)

The obtained concealer was uniform without lumps or the like, and was smooth, spread well, was lightweight and even in color, and gave a feeling of full coverage when applied to the skin.

### Example 8 (sunscreen)

| | | |
|---|---|---|
| **(1)** | **Water** | **63.91** |
| **(2)** | **Edeta BD manufactured by BASF SE: disodium edetate** | **0.10** |
| **(3)** | **1,3-butanediol (reagent)** | **3.00** |
| **(4)** | **Pemulen TR-1 manufactured by Lubrizol Corporation: (acrylates/alkyl (C10-30) acrylate) crosspolymer** | **0.20** |
| **(5)** | **SilFonn HydroFlex*: polyacrylate-56** | **1.00** |
| **(6)** | **COSMOL 82 manufactured by The Nisshin OilliO Group, Ltd.: sorbitan sesquioleate** | **0.40** |
| **(7)** | **NONION ST-60 manufactured by NOF CORPORATION: polyoxyethylene sorbitan monostearate** | **0.80** |
| **(8)** | **Cetiol AB manufactured by BASF SE: alkyl (C12-C15) benzoate** | **2.00** |
| **(9)** | **(A) 1,1,1,3,5,5,5-heptamethyl-3-dodecyltrisiloxane (synthetic product)** | **2.00** |
| **(10)** | **(C) Element14 PDMS 10-JC* (surface tension 20.1 mN/m, viscosity 10 cSt): polydimethylsiloxane molecular weight 1200 (calculated value)** | **1.00** |
| **(11)** | **(B) SI01-2 TiO₂ MT-500SA manufactured by DAITO KASEI KOGYO CO., LTD.: hydrophobically surface-treated titanium oxide** | **3.00** |
| **(12)** | **Uvinul A Plus manufactured by BASF SE: diethylamino hydroxybenzoyl hexyl benzoate** | **7.00** |
| **(13)** | **Uvinul MC 80 manufactured by BASF SE: 2-ethylhexyl paramethoxycinnamate** | **7.00** |
| **(14)** | **Uvinul T 150 manufactured by BASF SE: ethylhexyl triazone** | **4.00** |
| **(15)** | **KALCOL 6850 manufactured by Kao Corporation: cetearyl alcohol** | **1.00** |
| **(16)** | **NONION SP-60RP manufactured by NOF CORPORATION: sorbitan stearate** | **3.00** |
| **(17)** | **AMP ULTRA PC 2000 manufactured by ANGUS CHEMICAL COMPANY: 2-amino-2-methyl-1-propanol** | **0.09** |
| **(18)** | **Phenoxy ethanol (reagent)** | **0.50** |

| | | |
|---|---|---|
| **All the numerical values are expressed as mass%, and total up to 100 mass%.** **Those followed by an asterisk (*) are manufactured by Momentive Performance Materials Inc.** | | |

### (Preparation method)

The above components (1) to (5) and components (6) to (16) were each mixed until they became uniform, and heated to 80°C. The mixed liquid of the components (6) to (16) was added to the mixed liquid of the components (1) to (5) under stirring, and after the pH was made to 6 to 7 with triethanolamine, stirring was continued to obtain a uniform liquid. After cooling to 40°C, the components (17) and (18) were added and stirred to obtain an oil-in-water type sunscreen.

### (Evaluation)

The obtained sunscreen was uniform without lumps or the like, and was smooth, spread well, and provided an even coating when applied to the skin.

### Example 9 (mascara)

| | | |
|---|---|---|
| **(1)** | **Water** | **34.30** |
| **(2)** | **HARMONIE Luxe 4 powder*: silica** | **5.00** |
| **(3)** | **PVP K30 manufactured by Ashland Inc.: polyvinylpyrrolidone** | **2.00** |
| **(4)** | **Natrosol Plus manufactured by Ashland Inc.: hydroxyethyl cellulose** | **1.00** |
| **(5)** | **Triethanolamine (reagent)** | **1.00** |
| **(6)** | **Methylparaben (reagent)** | **0.30** |
| **(7)** | **Dissolvine NA2-S manufactured by Lion Corporation: EDTA 2Na** | **0.10** |
| **(8)** | **(B) SIH-2 BLACK No. 710P manufactured by DAITO KASEI KOGYO CO., LTD.: surface-untreated black pigment** | **10.00** |
| **(9)** | **SilForm HydroFlex*: polyacrylate-56, water** | **7.20** |
| **(10)** | **LUNAC S-98 manufactured by Kao Corporation: stearic acid** | **4.50** |
| **(11)** | **EMALEX GMS-F manufactured by NIHON EMULSION Co., Ltd.: glyceryl stearate** | **2.00** |
| **(12)** | **(A) 1,1,1,3,5,5,5-heptamethyl-3-dodecyltrisiloxane (synthetic product)** | **2.00** |
| **(13)** | **(C) Element14 PDMS 10-JC* (surface tension 20.1 mN/m, viscosity 10 cSt): polydimethylsiloxane molecular weight 1200 (calculated value)** | **2.00** |
| **(14)** | **Purified Beeswax CY-100 manufactured by YOKOZEKI OIL & FAT INDUSTRIES CO., LTD.** | **3.00** |
| **(15)** | **Purified Carnauba wax R-100 manufactured by YOKOZEKI OIL & FAT INDUSTRIES CO., LTD.** | **4.50** |
| **(16)** | **Lanohydro manufactured by Croda International Plc: hydrogenated lanolin** | **1.00** |
| **(17)** | **Propylparaben (reagent)** | **0.10** |
| **(18)** | **Avalure AC 120 Polymer manufactured by Lubrizol Corporation: acrylates copolymer** | **20.00** |

| | | |
|---|---|---|
| **All the numerical values are expressed as mass%, and total up to 100 mass %.** **Those followed by an asterisk (*) are manufactured by Momentive Performance Materials Inc.** | | |

### (Preparation method)

The above components (1) to (3) were stirred well to be a uniform liquid and then heated to 82 to 85°C, and the components (4) to (9) were added and stirred until they became uniform. The components (10) to (17) were stirred at 87°C until they became uniform, added to the mixed liquid of the components (1) to (9), stirred until they became uniform, and cooled to 60°C, and the component (18) was added thereto, stirred until they became uniform, and cooled to room temperature to obtain a mascara.

### (Evaluation)

The obtained mascara was uniform without lumps or the like, and was smooth, spread well, and provided an even coating when applied to the skin.

### Example 10 (oil-in-water type body cream)

| | | |
|---|---|---|
| **(1)** | **Water** | **76.50** |
| **(2)** | **HARMONIE Luxe 4 powder*: silica** | **9.50** |
| **(3)** | **(B) SIH TiO₂ R250 manufactured by DAITO KASEI KOGYO CO., LTD.: hydrophilically surface-treated titanium oxide** | **0.50** |
| **(4)** | **Silsoft EAU Microgel*: polysilicone-34, isononyl isononanoate, water** | **3.00** |
| **(5)** | **(C) Element14 PDMS 5-JC* (surface tension 19.7 mN/m, viscosity 5 cSt): polydimethylsiloxane molecular weight 680 (calculated value)** | **5.00** |
| **(6)** | **(A) 1,1,1,3,5,5,5-heptamethyl-3-dodecyltrisiloxane (synthetic product)** | **3.00** |
| **(7)** | **Star Shea Butter Refined manufactured by Nikko Chemicals Co., Ltd.: shea butter** | **2.00** |
| **(8)** | **Phenoxy ethanol (reagent)** | **0.50** |

| | | |
|---|---|---|
| **All the numerical values are expressed as mass%, and total up to 100 mass%.** **Those followed by an asterisk (*) are manufactured by Momentive Performance Materials Inc.** | | |

### (Preparation method)

The above components (1) to (3) and components (4) to (7) were each stirred at 80°C until they became uniform, and the mixed liquid of the components (4) to (9) was added to the mixed liquid of the components (1) to (3), stirred until they became uniform, and cooled to room temperature to obtain an oil-in-water type body cream.

### (Evaluation)

The obtained body cream was smooth, spread well, and provided an even coating when applied to the skin.

### Industrial Applicability

The cosmetic preparation of the present invention can be in any of liquid formulations, powder formulations, solid formulations, and emulsion formulations, and can be utilized as a skin cosmetic preparation selected from foundations, cosmetic primers, sunscreens, lipsticks, eye shadows, nail polishes, and face powders.

## Claims

1. A cosmetic preparation comprising the following components (A), (B), and (C):
(A) one or two or more selected from 1,1,1,3,5,5,5-heptamethyl-3-alkyl (C10-14) trisiloxanes;
(B) fine metal oxide particles; and
(C) a silicone oil with a number average molecular weight of 400 or more and less than 30,000 (excluding component (A)).

2. The cosmetic preparation according to claim 1, wherein the component (A) is any one component selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

3. The cosmetic preparation according to claim 1, wherein the component (A) is any of combinations of components (A-1) and (A-2), components (A-1) and (A-3), and components (A-2) and (A-3) selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

4. The cosmetic preparation according to claim 1, wherein the component (A) comprises three components (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

5. The cosmetic preparation according to claim 1, wherein the fine metal oxide particles of the component (B) are selected from fine iron oxide particles, fine titanium oxide particles, fine zinc oxide particles, fine zirconium oxide particles, fine cerium oxide particles, or combinations of these.

6. The cosmetic preparation according to claim 1, wherein the fine metal oxide particles of the component (B) are selected from fine iron oxide particles, fine titanium oxide particles, fine zinc oxide particles, fine zirconium oxide particles, fine cerium oxide particles, or combinations of these, and are hydrophilically or hydrophobically treated.

7. The cosmetic preparation according to claim 1, wherein the silicone oil of the component (C) is represented by the following composition formula:
RₐSiO_{(4-a)/2}
wherein R is a substituted or unsubstituted monovalent hydrocarbon group, the monovalent hydrocarbon groups may be the same or different, and a represents a positive number less than 4.

8. The cosmetic preparation according to claim 1, wherein the silicone oil of the component (C) has a surface tension of 30 mN/m or less.

9. The cosmetic preparation according to claim 1, wherein the component (C) is selected from polydimethylsiloxanes, polydimethylsiloxane-polydiphenylsiloxane copolymers, polymethyltrimethylsilylsiloxane-polydiphenylsiloxane copolymers, trimethylsilyl-terminated mono or polytrimethylsilylphenylsiloxanes, polyether-modified oils, or combinations of these.

10. The cosmetic preparation according to claim 1, wherein a mass ratio of a content of the component (A) to a content of the component (C) ((A)/(C)) is in a range of 0.1 to 5.

11. The cosmetic preparation according to claim 1, wherein the preparation comprises the components (A), (B), and (C) in an amount of 3 to 85 mass% in total.

12. The cosmetic preparation according to any one of claims 1 to 11, wherein the preparation is in any of liquid formulations, powder formulations, solid formulations, and emulsion formulations.

13. The cosmetic preparation according to any one of claims 1 to 11, wherein the preparation is a skin cosmetic preparation selected from foundations, cosmetic primers, sunscreens, lipsticks, eye shadows, nail polishes, and face powders.
